(19) Europäisches Patentamt

European Patent Office

Office européen des brevets

(11) **EP 2 769 727 B1**

(12) **EUROPEAN PATENT SPECIFICATION**

(45) Date of publication and mention of the grant of the patent:
**18.05.2016 Bulletin 2016/20**

(51) Int Cl.:
*A61K 31/708* *(2006.01)*  *A61K 31/7076* *(2006.01)*
*A61K 31/7072* *(2006.01)*  *A61K 31/7068* *(2006.01)*
*A61P 35/00* *(2006.01)*  *A61P 35/02* *(2006.01)*

(21) Application number: **12842329.0**

(22) Date of filing: **16.10.2012**

(86) International application number:
**PCT/CN2012/001392**

(87) International publication number:
**WO 2013/056510 (25.04.2013 Gazette 2013/17)**

(54) **DEOXYNUCLEOSIDE/NUCLEOSIDE COMBINATION FOR USE IN THE TREATMENT OF TUMORS**

DESOXYNUKLEOSID-/NUKLEOSID-KOMBINATION ZUR VERWENDUNG BEI DER BEHANDLUNG VON TUMOREN

COMBINAISON DÉSOXYNUCLÉOSIDE/NUCLÉOSIDE POUR UTILISATION DANS LE TRAITEMENT DE TUMEURS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.10.2011 CN 201110314309**

(43) Date of publication of application:
**27.08.2014 Bulletin 2014/35**

(73) Proprietor: **Zhang, Shizhuang**
**Weifang, Shandong 261031 (CN)**

(72) Inventors:
  • **ZHANG, Shizhuang**
   **Weifang**
   **Shandong 261031 (CN)**
  • **CHENG, Xin**
   **Weifang**
   **Shandong 261053 (CN)**
  • **GAO, Zhiqin**
   **Weifang**
   **Shandong 261053 (CN)**
  • **HAN, Ming**
   **Weifang**
   **Shandong 261053 (CN)**

(74) Representative: **Sun, Yiming**
**HUASUN Patent- und Rechtsanwälte**
**Friedrichstraße 33**
**80801 München (DE)**

(56) References cited:
**WO-A2-99/02143**      **CN-A- 102 172 359**
**CN-A- 102 172 359**     **CN-A- 102 499 937**
**GB-A- 1 270 049**       **US-A1- 2001 044 421**
**US-A1- 2002 198 183**

• **KIM K T ET AL: "The effect of pyrimidine nucleosides on adenosine-induced apoptosis in HL-60 cells", JOURNAL OF CANCER RESEARCH AND CLINICAL ONCOLOGY 1998 DE, vol. 124, no. 9, 1998, pages 471-477, XP002728120, ISSN: 0171-5216**
• **LOWE J K ET AL: "Inhibition of growth rate and deoxynucleoside triphosphate concentrations in cultured leukemia L1210 cells", MOLECULAR PHARMACOLOGY, vol. 12, no. 1, 1976, pages 177-184, XP009179465, ISSN: 0026-895X**
• **COHEN J D ET AL: "Deoxyribonucleoside triphosphate pools and thymidine chemosensitization in human T-cell leukemia", LEUKEMIA RESEARCH, NEW YORK,NY, US, vol. 17, no. 2, February 1993 (1993-02), pages 167-174, XP026353256, ISSN: 0145-2126 [retrieved on 1993-02-01]**
• **FRIED J ET AL: "Cytotoxic and cytokinetic effects of thymidine, 5-fluorouracil, and deoxycytidine on HeLa cells in culture", CANCER RESEARCH, vol. 41, no. 7, 1981, pages 2627-2632, XP002728121, ISSN: 0008-5472**

**EP 2 769 727 B1**

**(Cont. next page)**

- ROSS D D ET AL: "Effects of deoxynucleosides on cultured human leukemia cell growth and deoxynucleotide pools.", CANCER RESEARCH NOV 1981, vol. 41, no. 11 Pt 1, November 1981 (1981-11), pages 4493-4498, XP002728122, ISSN: 0008-5472

- ZHEN Y S ET AL: "Effects of acivicin and dipyridamole on hepatoma 3924A cells.", CANCER RESEARCH APR 1983, vol. 43, no. 4, April 1983 (1983-04), pages 1616-1619, XP055132458, ISSN: 0008-5472

**Description**

**TECHNICAL FIELD**

The invention relates to the combination of deoxynucleoside and a nucleoside for use in the treatment of tumors.

**BACKGROUND TECHNOLOGY**

Nucleoside is linked by base and pentose (ribose or deoxyribose), namely, it is a compound linked by N-9 of purine or N-1 of pyrimidine and C-1 of ribose or deoxyribose via β glycosidic bond, including ribonucleoside and deoxyribonucleoside. Ribonucleoside is nucleoside of RNA, and mainly includes adenosine, guanosine, cytidine, and uridine; deoxyribonucleoside (hereafter referred to as deoxynucleoside) is nucleoside of DNA, and mainly includes deoxyadenosine, deoxyguanosine, deoxy thymidine, and deoxycytidine. Nucleoside can be prepared from the hydrolysis of nucleic acid. Pyridine solution, alumina, or enzymatic hydrolysis of RNA can be used to obtain ribonucleoside; alumina or enzymatic hydrolysis of DNA can be used to obtain deoxyribonucleoside. Nucleoside can also be chemically synthesized. Suitably protected ribose or deoxyribose condensed with base derivatives, the corresponding ribonucleoside and deoxyribonucleoside shall be obtained.

Ribonucleoside is a compound covalently bonded by purine (except thymidine) or pyrimidine and ribose molecule, which mainly includes adenosine, guanosine, cytidine and uridine. Adenine nucleoside is referred to as adenosine, chemical name: 9-PD-ribofuranosyl adenine, CAS NO.: 58-61-7, MF: C10H13N5O4, MW: 267.24.Guanosine, chemical name: 9-PD-furan guanine nucleoside, CAS No.:118-00-3, MF: C1GH13N5O5, MW: 283.24. Cytidine, chemical name: 1-β-D-ribofuranosyl-cytosine, CAS NO.: 65-46-3, MF: C9H13N3O5, MW: 243.22. Uridine, chemical name: 1-β-D- ribofuranosyl uracil, CAS NO.: 58-96-8, MF: C9H12N2O6, MW: 244.20.

Deoxyribonucleoside is a compound linked by N-9 of purine (adenine, guanine) or N-1 of pyrimidine base (cytosine, thymine) and C- 1 of 2-deoxy-D- ribose via β glycosidic bond, the main deoxynucleoside includes deoxyadenosine, deoxyguanosine, deoxycytidine, and thymidine (referred to as thymidine).Deoxyadenosine, i.e., 2'-adenine deoxyribonucleoside, chemical name: 9-β-D-2'- deoxy nucleoside furan adenine, which is a compound linked by N-9 of adenine and C-1 of 2-deoxy-D- ribose via β glycosidic bond, CAS NO.:958-09-8, MF: C1 () H13N5O3, MW: 251.24. Deoxyguanosine, namely, guanine deoxyribonucleoside, chemical name: 9-β-2 furan deoxynucleoside guanine, which is a compound linked by N-9 of guanine and C-1 of 2-deoxy-D- ribose via β glycosidic bond, CAS NO.: 961-07-9, MF: C10H13N5O4, MW: 267.24. Deoxycytidine, namely, 2'-deoxycytidine nucleoside, chemical name: 1-β-2'- furan deoxynucleoside cytosine, which is a compound linked by N-1 of cytosine and C-1 of 2-deoxy-D-ribose via β glycosidic bond, CAS NO.: 951-77- 9, MF: C9H13N3O4, MW: 227.22. Thymidine, namely, 2'-deoxythymidine, or β-thymidine, chemical name: 9-PD-furan nucleoside thymidine, CAS No.: 50-89-5, MF: C10H14N2O5, MW: 242.023.

Monophosphate deoxyribonucleoside is a structural fragment of desoxyribonucleic acid DNA, nucleoside and its derivatives have good physical activity, and they are important raw materials of genetic medicine and genetic engineering research. Such as 2-chlorodeoxyadenosine can be used for the treatment of leukemia, 2' deoxy, 3' deoxyadenosine, 2 deoxy adenosine and 3 deoxyadenosine extracted from the composite cordycepin can inhibit tumor significantly, deoxy-freecyclic guanosine (dixiluowei) has significant antiviral activity, 2 deoxycytidine (Tashi cytidine) are commonly used medicaments to treat AIDS clinically. So nucleoside analogs are very good intermediates for many anti-viral, anti-tumor, anti-AIDS medicaments. But the combination of deoxynucleoside and/or with other nucleoside used in anti- tumor areas has not been reported yet.

**SUMMARY OF THE INVENTION**

The invention is aimed to provide a combination of deoxynucleosides and a nucleoside for use in the treatment of tumors, wherein the deoxynucleosides comprises deoxyadenosine and deoxyguanosine; and wherein the nucleoside is a ribonucleoside selected from adenosine, guanosine, cytidine or uridine. , The combination can achieve significantly improved anti-tumor effect then single use, reduce side effects and delay the occurrence of medicament resistance.

Technical program of the invention:

The deoxynucleoside/nucleoside combination for use in the treatment of tumor, the said deoxynucleosides include deoxyadenosine, deoxyguanosine; the said ribonucleoside includes adenosine, guanosine, cytidine, and uridine.

Wherein the said deoxynucleosides include deoxyadenosine and deoxyguanosine; the said ribonucleoside includes one or more of adenosine, guanosine, cytidine, and uridine.

Preferably, the said ribonucleoside includes one or two of adenosine, guanosine, cytidine, uridine, and the selected deoxynucleoside shall be different from ribonucleoside.

Further, each component has the same number of moles.

The said tumor includes stomach cancer, lung cancer, liver cancer, colon cancer, breast cancer, leukemia, reproductive system cancer and many other common malignant tumors.

The study found that: different combination of nucleosides medicament shall decrease medicament concentration in the blood than the single medicament, and thus decrease the side effects. At the same time, multi-medicament treatment can delay the occurrence of tumor resistance. By a combination of medication, it can reduce the dosage of each component compared with the single medicament, and the absolute concentration of each specific nucleoside shall decrease, and side effects in medicament shall also decrease; meanwhile, two or more chemotherapeutic agents in combination therapy may greatly reduce the occurrence of resistance. Compared with deoxynucleoside and another nucleoside medicament, the combination of deoxynucleoside and two other nucleosides shall greatly reduce resistance; compared to the combination of deoxynucleoside and other four nucleosides medicament, the combination of deoxynucleoside and two other nucleosides shall be easy to use and prepare while reducing the resistance.

After the combination of deoxynucleoside and the ribonucleosides, it shall be used in preparation of the above tumor medicament, which can be directly produced into an injection for injection use; the injection may be conventional, but also various types of sustained-release injections.

Different tumors have different nucleotide metabolism and combination characteristics, and each nucleoside shall have different inhibition strength on different tumor cells. The study found that the anti-tumor effect of the purine nucleoside is stronger than pyrimidine nucleoside, and metabolites of purine nucleoside, namely, inosine (i.e., inosine) also has some anti-tumor effect; the anti-tumor effect of deoxynucleoside with the same base is stronger than non-deoxynucleoside. Deoxyadenosine, deoxyguanosine, deoxycytidine and their corresponding non-deoxynucleoside (i.e., adenosine, guanosine, cytidine, uridine) has an oxygen atom difference in the molecule, the molecular weight is less than 16D, and can show anti-tumor activity above a concentration of 0.03mmol/L, and vitro inhibition rate can reach over 80% above a concentration of 0.4mmol/L. The antitumor effects of deoxynucleoside are significant, single deoxyadenosine treatment of 15% cancer tumor size is completed alleviated, the effective rate shall be more than 91.0%, and the use of dose is low, which shall be 4.0 ~ 35.0 mmol/Kg/day, continuous slow intravenous drip.

Advantages of the invention:

1. The combination of deoxyadenosine and ribonucleoside is for use in the treatment of many common tumors medicament, such as gastric cancer, lung cancer, liver cancer, colon cancer, breast cancer, reproductive system cancer and others. It has obvious anti-tumor effect, as hole as high efficiency, broad-spectrum and low toxic characteristics; and no cytotoxic effect on normal cells is the biggest advantage of this invention.

2. The combination of deoxyadenosine and ribonucleoside medicament has lower concentration in the blood than single medicament, thereby reducing the side effects;

3. The combination of deoxyadenosine and ribonucleoside medicament can delay the occurrence of tumor resistance.

## DETAILED EMBODIMENTS

Hereinafter, the preferred embodiments of the invention will be described, it should be understood that the preferred embodiments described herein are used for illustrating and explaining the invention only, which are not intended to limit to the invention.

### Example 1

Tumor inhibition tests of deoxyadenosine and ribonucleoside

I. Test method

The human hepatoma Bel-7402 tumor cells and human lung cancer cells PG (provided by Shanghai Institute of Cell, Chinese Academy of Sciences), which are cultured by RPMI 1640 medium containing 10 % fetal bovine serum, cells in logarithmic growth phase are seeded in 96-hole plates with 3x103 hole after digested with 0.25% trypsin, placed in 37°C, 5%C02 incubator for 24 hours, each set eight holes. The test groups are added 60uL nucleoside solution with the total concentration of 10.0mmol/L, supplemented culture medium to each hole 200uL, 5-FU treatment group of the control group has the same deoxyadenosine amount (mmol/L), which is diluted with serum-free medium, and filtered by 0.22jxm sterile micropore membrane. In the 48 hours of measurement time, a microscope is inverted to observe and photograph cell morphology, and then each hole is added into 20 uL of 5mg/mL MTT solution, placed in 37°C, 5% C02 incubator and cultured for 4 hours. After the termination of culture, suck and abandon the cultured supernatant, each hole is added to 150pLDMSO, shock for 10 minutes to fully dissolve crystals, and measure absorbance degrees at 570mn with automatic microplate reader (Thermo, Model MULTISKANMK3). Each plate shall set 8 holes, each test shall be repeated three times, and the data in Table 1 are the average of three tests number. In the subsequent embodiments, the data is calculated in the same method with the present embodiment.

Tumor cell growth inhibition rate is calculated as follows:

$$\text{Tumor cell viability } (\%) = \text{actual OD value of the dosing hole } / \text{OD value of the negative control hole;}$$

$$\text{Tumor cell growth inhibition rate } (\%) = 100\% \text{-cell survival.}$$

**Table 1 48 Hours Inhibition Rate of Various nucleoside on 7402 and PG Tumor Cells (%)**

| Group | A | G | C | U | dA | dG | dC | T | 5-FU |
|---|---|---|---|---|---|---|---|---|---|
| Hepatic cancer | 86 | 83 | 65 | 49 | 96 | 95 | 86 | 87 | 91 |
| Arsine cancer | 84 | 87 | 53 | 52 | 94 | 96 | 84 | 88 | 90 |
| (Table: A, G, C, U shall represent adenosine, guanosine, cytidine and uridine, dA, dG, dC, | | | | | | | | | |

T shall represent deoxyadenosine, deoxyguanosine, deoxycytidine and thymidine)

II. Results analysis

From Table 1 it can be seen that:

1. 1 Both deoxyadenosine and non- deoxyadenosine have anti-tumor effects;

2. The antitumor effect of purine nucleoside is stronger than pyrimidine nucleoside;

3. The antitumor effect of deoxyadenosine is stronger than non-deoxyadenosine.

**Example 2**

Tumor inhibition tests of the combination of one deoxyadenosine and one nucleoside, single medicament of the same kind deoxyadenosine and nucleoside

I. Test method

Various combination of deoxyribonucleoside and ribonucleoside and the control group (5-FU) tests are shown in Table 2. Treatment is applied in human colon cancer cell line HT-29 tumor cells (provided by Institute of Basic Medical, Chinese Academy of Sciences), methods, and conditions are the same as in Example 1. The total concentration of nucleoside, single nucleoside concentration and 5-FU concentration shall be the same with the Example 1.

**Table 2 Inhibition Rate of the Combination of Deoxynucleoside / Nucleoside on HT-29 Cells (%)**

| dA Group | dA+G | dA+C | dA+U | dA+A | | dA+dC | dA | 5-FU |
|---|---|---|---|---|---|---|---|---|
| 48 Hour | 95 | 86 | 87 | 93 | | 92 | 86 | 91 |
| dG Group | dG+G | dG4C | dG+U | dG+A | dG+dA | dG+dC | dG | 5-FU |
| 48 Hour | 93 | 87 | 85 | 96 | 95 | 91 | 91 | 93 |

(continued)

| dA Group | dA+G | dA+C | dA+U | dA+A | | dA+dC | dA | 5-FU |
|---|---|---|---|---|---|---|---|---|
| dC Group | dC+G | dC+C | dC+U | dC+A | | | dC | 5-FU Group |
| 48 Hour | 91 | 80 | 83 | 90 | | | 89 | 96 |
| T Group | T+G | T+C | T+U | T+A | T+dA | T+dG | T | 5-FU Group |
| 48 Hour | 92 | 82 | 80 | 91 | 87 | 86 | 85 | 95 |

II. Results analysis

As can be seen from Table 2, the antitumor effects of the combination of deoxynucleoside and nucleoside are stronger than the inhibition rate of single nucleotide; due to single nucleotide concentrations is reduced by 50 %, thus its side effects are also reduced as a result.

**Example 3**

Tumor inhibition tests of the combination of two deoxynucleoside, single deoxynucleoside, single ribonucleoside, and the control group (5-FU) on the human hepatoma Bel-7402.

I. Test method:

Various combination of deoxyribonucleoside and ribonucleoside and the control group (5-FU) tests are shown in Table 3. Treatment is applied in human hepatoma Bel-7402 tumor cells (provided by Institute of Basic Medical, Chinese Academy of Sciences), methods, and conditions are the same as in Example 1. The total concentration of nucleoside, single nucleoside concentration and 5-FU concentration shall be the same with the Example 1.

**Table 3 The Inhibition Rate of Different Combination of Deoxyribonucleosides on 7402 (%)**

| | dG+A+T | dG | A+T | 5-FU |
|---|---|---|---|---|
| 48H | 92 | 87 | 86 | 91 |
| 72H | 95 | 93 | 94 | 95 |

II. Results analysis

As can be seen from Table 3, the combination of one deoxynucleoside and two nucleosides Significant has obvious inhibition rate, which is higher than the inhibition rate of single deoxynucleoside or only the combination of two nucleotides, and clinical findings of its toxicity side effect is lower.

**Example 4**

Tumor inhibition tests of the combination of one deoxynucleoside and two nucleoside (one deoxidation and one non-deoxidation), and the same kind of single deoxyribonucleoside, single two ribonucleoside alone and the control group (5-FU) on the human gastric cancer BGC823 cell lines.

I. Test method

Various combination of deoxyribonucleoside and ribonucleoside and the control group (5-FU) tests are shown in Table 4. Treatment is applied in human gastric cancer cell lines BGC823 tumor cells (provided by Institute of Basic Medical, Chinese Academy of Sciences), methods, and conditions are the same as in Example 1. The total concentration of nucleoside, single nucleoside concentration and 5-FU concentration shall be the same with the Example 1.

**Table 4 The Inhibition Rate of Different Combination of Deoxyribonucleosides on BGC823 (%)**

| | dA+dG+C | dG+C | dA | dG+dC+U | dC+U | dG | dC+T+A | 5-FU |
|---|---|---|---|---|---|---|---|---|
| 48H | 95 | 90 | 89 | 90 | 83 | 85 | 88 | 91 |

(continued)

|  | dA+dG+C | dG+C | dA | dG+dC+U | dC+U | dG | dC+T+A | 5-FU |
|---|---|---|---|---|---|---|---|---|
| 72H | 98 | 93 | 91 | 94 | 89 | 90 | 91 | 95 |

II. Results analysis

It can be seen from Table 4 that:

1. The tumor inhibition effects of one deoxynucleoside, the combination of one deoxidation and one non-deoxidation nucleoside, the combination of two deoxynucleoside and one non- deoxynucleoside are improved in order, wherein, due to dA + dG + C has two kinds of deoxy purine nucleoside, it has the most obvious inhibition effect on BGC823 gastric cancer cell lines;

2. The nucleoside combination does not only decreases inhibition effects on tumor, but also increase inhibition effects on tumor.

## Example 5

Tumor inhibition tests of the combination of two deoxynucleoside and one nucleoside, and the same kind of single two deoxyribonucleoside, single one ribonucleoside alone and the control group (5-FU) on the human breast cancer cell lines Bcap-37.

I. Test method

Various combination of deoxyribonucleoside and ribonucleoside and the control group (5-FU) tests are shown in Table 5. Treatment is applied in human breast cancer cells Bcap-37 tumor cells (provided by Institute of Basic Medical, Chinese Academy of Sciences), methods, and conditions are the same as in Example 1. The total concentration of nucleoside, single nucleoside concentration and 5-FU concentration shall be the same with the Example 1.

**Table 5 The Inhibition Rate of Two Deoxyribonucleoside and One Ribonucleoside on Bcap-37 (%)**

|  | dA+dG +C | dA+dG | C | dG+dC+U | dG+dC | U | dC+T+A | 5-FU |
|---|---|---|---|---|---|---|---|---|
| 48H | 93 | 90 | 65 | 89 | 80 | 58 | 85 | 92 |
| 72H | 96 | 93 | 71 | 92 | 85 | 67 | 90 | 95 |

III. Results analysis

It can be seen from the Table 5 that:

1. The tumor inhibition effects of one deoxynucleoside, the combination of two deoxidation, and the combination of two deoxynucleoside and one non-deoxynucleoside are improved in order, wherein, the combination of two deoxynucleoside and one ribonucleoside has the most obvious inhibition effect on Bcap-37;

2. Changes of the combination of nucleoside and other inhibition rate are relevant to tumor inhibition characteristics of nucleoside.

## Example 6

Tumor inhibition tests of the combination of two deoxynucleoside and two non-deoxynucleoside, single two deoxyribonucleoside, single two ribonucleoside alone and the control group (5-FU) on the human cervical cancer cell line (Hela).

I. Test method

Various combination of deoxyribonucleoside and ribonucleoside and the control group (5-FU) tests are shown in Table 6. Treatment is applied in human cervical cancer cell line (Hela) (provided by Institute of Basic Medical, Chinese Academy of Sciences), methods, and conditions are the same as in Example 1. The total concentration of nucleoside, single nucleoside concentration and 5-FU concentration shall be the same with the Example 1.

**Table 6 The Inhibition Rate of Two Deoxyribonucleoside Combination and Two Non-deoxyribonucleoside Combination on Hela (%)**

| | dA+dG +C+U | dA+dG | c+u | dG+dC +A+G | dG+dC | A+G | dC+T+ G4C | 5-FU |
|---|---|---|---|---|---|---|---|---|
| 48H | 87 | 93 | 60 | SO | 85 | 80 | 78 | 92 |
| 72H | 92 | 97 | 67 | 84 | 90 | 87 | 82 | 96 |

II. Results analysis

It can be seen from the Table 6 that:

1. Compared to the combination of 3 kinds, advantages of the combination of 4 kinds are not obvious;

2. Hela cells show that antitumor effect of deoxynucleoside is superior to non-deoxynucleoside.

**Example 7**

Tumor inhibition tests of the combination of two deoxynucleoside and other two deoxynucleoside, single two deoxyribonucleoside alone and the control group (5-FU) on the human glioma cell line SHG-44.

I. Test method

Various combination of deoxyribonucleoside and the control group (5-FU) tests are shown in Table 7. Treatment is applied in human glioma SHG-44 tumor cells (provided by Institute of Basic Medical, Chinese Academy of Sciences), methods, and conditions are the same as in Example 1. The total concentration of nucleoside, single nucleoside concentration and 5-FU concentration shall be the same with the Example 1.

**Table 7 The Inhibition Rate of Four Deoxyribonucleoside Combination and Two Deoxyribonucleoside Combination on SHG-44 (%)**

| | dA+dG +dC+T | dA+dG | dG+ dC | dC+T | T+ dA | 5-FU |
|---|---|---|---|---|---|---|
| 48H | 84 | 94 | 80 | 74 | 79 | 91 |
| 72 H | 90 | 98 | 86 | 81 | 85 | 96 |

II. Results analysis

It can be seen from the Table 7 that: compared to two deoxyribonucleoside combination and four deoxynucleoside combination, the anti-tumor effect of two deoxy purine nucleoside is the strongest, the effect of four deoxynucleotide combination is the second strongest, and the effect of pyrimidine nucleoside is the smallest.

**Example 8**

Tumor inhibition tests of the combination of two deoxynucleoside and one non-deoxynucleoside with a different ratio on human pancreatic cancer cell line PANC-1.

I. Test method

The inhibitory test with the combination of two deoxynucleoside (dA + dG) and one deoxynucleoside (C) and the control group (5-FU), as shown in Table 8. Treatment is applied in human glioma SHG-44 tumor cells (provided by Institute of Basic Medical, Chinese Academy of Sciences), methods, and conditions are the same as in Example 1. The total concentration of nucleoside, single nucleoside concentration and 5-FU concentration shall be the same with the Example 1.

Wherein, A1 medicament ratio is 1:1:1, A2 medicament ratio is 2:2:1, A3 medicament ratio is 1:1:2, A4 medicament ratio is 2:1:2, A5 medicament ratio is 3: 3: 1, A6 medicament ratio is 1:1:3, and A7 medication ratio is 3:1:3.

**Table 8 The Inhibition Rate of Two Deoxyribonucleoside (dA+ dG) and One Non-deoxyribonucleoside (C) on PANC-1 (%)**

|       | A1 | A2 | A3 | A4 | A5 | A6 | A7 | 5-FU |
|-------|----|----|----|----|----|----|----|------|
| 48H   | 86 | 91 | 80 | 82 | 93 | 71 | 88 | 93   |
| 72 H  | 91 | 96 | 85 | 86 | 97 | 86 | 91 | 95   |

II. Results analysis

It can be seen from the Table 8 that: the optimal anti-tumor effect is achieved when the medication ratio of two deoxyribonucleoside and one non-deoxyribonucleoside is A5 (medication ratio is: dA: dG: C 3: 3: 1). Meanwhile in the preparation of clinical tumor medicament, it shall take into account the feasibility and safety of clinical medicine.

Based on above-described embodiments of human tumor cells inhibition tests, it has been found compared with single medication, the combination of deoxynucleoside and nucleoside had no cytotoxic effects.

Finally, it should be noted that: the above are only preferred embodiments of the invention, not intended to limit to the invention. Although the foregoing reference embodiments have made detailed description of the invention, the skilled person in the field can also modify the technical solutions described in example embodiments or make equivalent replacements to some technical features. Any modification, equivalent replacement, improvement, etc., within the spirit and principles of the invention should be included within the protection scope of the invention.

**Claims**

1. The combination of deoxynucleosides and a nucleoside for use in the treatment of tumors, wherein the deoxynucleosides comprises deoxyadenosine and deoxyguanosine; and wherein the nucleoside is a ribonucleoside selected from adenosine, guanosine, cytidine or uridine.

2. The combination for use according to claim 1, wherein the ribonucleoside includes one or more of adenosine, guanosine, cytidine, and uridine.

3. The combination for use according to claims 1 and 2, wherein the ribonucleoside includes two of adenosine, guanosine, cytidine, or uridine.

4. The combination for use according to claims 1 to 3, wherein each ribonucleoside and deoxynucleoside has the same number of mole.

5. The combination for use according to claims 1 to 4, wherein the tumors include gastric cancer, lung cancer, liver cancer, colorectal cancer, breast cancer, leukemia and reproductive system cancer.

**Patentansprüche**

1. Kombination aus Desoxynucleosiden und einem Nucleosid zur Verwendung bei der Behandlung von Tumoren, wobei die Desoxynucleosiden Desoxyadenosin und Desoxyguanosin umfasst; und wobei das Nucleosid ein aus Adenosin, Guanosin, Cytidin oder Uridin ausgewähltes Ribonucleosid ist.

2. Kombination zur Verwendung nach Anspruch 1, wobei das Ribonucleosid eines oder mehrere Elemente aus Adenosin, Guanosin, Cytidin und Uridin umfasst.

3. Kombination zur Verwendung nach den Ansprüchen 1 und 2, wobei das Ribonucleosid zwei Elemente aus Adenosin, Guanosin, Cytidin oder Uridin umfasst.

4. Kombination zur Verwendung nach den Ansprüchen 1 bis 3, wobei jedes Ribonucleosid und Desoxynucleosid die gleiche Anzahl von Molen aufweist.

**5.** Kombination zur Verwendung nach den Ansprüchen 1 bis 4, wobei die Tumoren Magenkrebs, Lungenkrebs, Leberkrebs, Dickdarmkrebs, Brustkrebs, Leukämie und Krebs des Fortpflanzungssystems umfassen.

**Revendications**

**1.** Combinaison de désoxynucléosides et un nucléoside pour utilisation dans le traitement de tumeurs, dans laquelle les désoxynucléosides comprennent la désoxyadénosine et la désoxyguanosine ; et dans laquelle le nucléoside est un ribonucléoside sélectionné parmi l'adénosine, la guanosine, la cytidine ou l'uridine.

**2.** Combinaison pour utilisation selon la revendication 1, dans laquelle le ribonucléoside comprend un ou plusieurs éléments parmi l'adénosine, la guanosine, la cytidine et l'uridine.

**3.** Combinaison pour utilisation selon les revendications 1 et 2, dans laquelle le ribonucléoside comprend deux éléments parmi l'adénosine, la guanosine, la cytidine ou l'uridine.

**4.** Combinaison pour utilisation selon les revendications 1 à 3, dans laquelle chaque ribonucléoside et désoxynucléoside présente le même nombre de moles.

**5.** Combinaison pour utilisation selon les revendications 1 à 4, dans laquelle les tumeurs comprennent le cancer de l'estomac, le cancer du poumon, le cancer du foie, le cancer colorectal, le cancer du sein, la leucémie et le cancer de l'appareil génital.